Europäisches Patentamt

European Patent Office

Office européen des brevets

(11) Publication number: **0 040 157**
**B1**

(12) **EUROPEAN PATENT SPECIFICATION**

(45) Date of publication of patent specification: **05.03.86**

(21) Application number: **81400760.5**

(22) Date of filing: **13.05.81**

(51) Int. Cl.⁴: **A 61 B 17/10, B 25 C 5/16**

(54) **Surgical stapling apparatus having independent staple feed and ejection capabilities.**

(30) Priority: **14.05.80 US 149656**

(43) Date of publication of application:
**18.11.81 Bulletin 81/46**

(45) Publication of the grant of the patent:
**05.03.86 Bulletin 86/10**

(84) Designated Contracting States:
**AT BE CH FR IT LI NL SE**

(56) References cited:
**GB-A- 201 107**
**US-A-2 090 831**
**US-A-2 966 681**

(73) Proprietor: **United States Surgical Corporation**
**150 Glover Avenue**
**Norwalk Connecticut 06850 (US)**

(72) Inventor: **Vey, Richard August**
**16 Buckingham Place**
**Norwalk Connecticut 06850 (US)**

(74) Representative: **Martin, Jean-Jacques et al**
**Cabinet REGIMBEAU 26, Avenue Kléber**
**F-75116 Paris (FR)**

Courier Press, Leamington Spa, England.

EP 0 040 157 B1

## Description

The present invention relates to surgical stapling mechanisms, in general, and to a surgical stapling mechanism having independent staple feed and ejection capabilities for use in a cartridge or instrument magazine, in particular.

Recently, surgical staple-carrying cartridges of the type used in conjunction with a permanent instrument and of the type incorporated into a disposable instrument have been designed which eliminate the requirement for the complex gearing once needed in the powering of a surgical stapling instrument. With these cartridges, the output shaft or thrust bar of the powering instrument need only have rectilinear thrust capabilities. The staples are advanced by means designed into the cartridges themselves. In U.S. Patent No. 3,618,842, the advancing pusher, integral with the cartridge, rotates a pair of staple-driving screws by means of cams formed in the rear portion of the screws. In U.S. Patent No. 3,638,847, the staples are driven forward by the interaction of pairs of opposing rachet teeth integral with the cartridge.

More recently, in U.S. Patent No. 3,650,453, the staple-carrying cartridge includes a flexible belt movably housed in the cartridge body. Staples are guided and advanced by association with spaced teeth on the flexible belt. The cartridge is equipped with an anvil integral with the cartridge body and a pusher which advances the staples and singly forms the same. Further improvements and modifications to the belt cartridge are disclosed in U.S. Patent No. 3,717,294. These prior cartridges are adapted to house staples which have a U-configuration prior to being formed around the anvil. The pusher and associated anvil are then adapted to form the staples into a rectangular configuration in which the points of the staples abut, or very nearly so.

The present invention relates to surgical staple feed and ejection apparatus fabricated as a disposable staple-carrying cartridge or magazine particularly suited for joining skin or fascia and adapted to be mounted on or formed as part of an instrument having rectilinear thrust capabilities. See U.S. Patent No. 4,204,623, which is incorporated by reference herein, for an example of a disposable staple-carrying cartridge used with a permanent instrument and examples of a disposable instrument including staple-carrying cartridge capabilities. Thus, it can be seen that the term "cartridge" as used herein is meant to include both a separate structure adapted to be mounted on an instrument and a structure formed as an integral part of an instrument.

In the earlier European patent application EP—A1—0 039 560, comprised in the state of the art according to Art. 54(3) of EPC, different types of stapler cartridges are disclosed. The cartridges basically comprise a main body having a rearward end for associating with a skin-stapling instrument and a forward end for the expulsion and formation of surgical staples. Defined within the main body is a staple-housing portion which is adaptable to store a plurality of surgical staples as a "total charge" in a linear or sequential array, one next to the other. The staple-housing portion contains a storage or guide track for orienting the surgical staples so that the crowns of the staples lie in substantially the same plane. A spring arrangement contained within the main body constantly urges the total charge of staples in a predetermined direction toward the forward end of the cartridge. An exit aperture provides a means by which the surgical staples may exit from the main body portion. Integral with the exit aperture is an anvil which is used to form the staples as they exit, one at a time, from the cartridge.

A delivery channel contained in the main body provides a passageway through which the staples move, one at a time, on their way toward the anvil. The delivery channel defines a plane, spaced from and substantially parallel to a plane defined by the crowns of the staples as they are stored in the staple-housing portion.

A short linear portion between the storage track and the delivery channel provides a path from the staple-housing portion to the delivery channel for the passage of the surgical staples.

Mounted within the main body for rectilinear movement is a pusher which responds to a force supplied by the stapling instrument to advance a staple through the delivery channel and out of the exit aperture for formation about the anvil.

The cartridge basically operates in the following manner. The staples, under the urging of the spring arrangement, move as a total charge or array along the storage track and through the short linear portion so that the forwardmost staple of the total charge is presented to the delivery channel. The short linear portion defines a plane which is substantially normal to both the plane defined by the staple crowns, when in the staple-housing portion, and the plane defined by the delivery channel. The forces which tend to cause the legs of the staples to project at right angles to the short linear portion will bias the legs of the staple against a staple guide surface in the delivery channel, so that the forwardmost staple of the total charge is reoriented from its storage position in the staple-housing portion to its position of intended use as it is presented to the delivery channel.

With the forwardmost staple positioned within the delivery channel, the pusher is activated through a force supplied by the skin-stapling instrument to move the pusher toward the forward end of the cartridge. As it moves, the pusher urges the forwardmost staple through the delivery channel and out of the exit aperture. The forwardmost staple is then presented to the anvil, and, through the continued forward movement of the pusher, is then formed about the anvil. The surgical stapling act is accomplished while the staple is being formed about the anvil.

It is a primary object of the present invention to provide a simplified stapling apparatus having independent staple feed and ejection capabilities, with an accurate reorientation of the forwardmost staple from its position of storage to its position of use.

It is another object of the present invention to provide a simplified disposable cartridge adapted to be mounted on a surgical stapling instrument having rectilinear thrust capabilities and being particularly suited for stapling skin and fascia.

It is a further object of the present invention to provide a staple-feeding and forming apparatus having a minimum number of movable parts and yet being both reliable and efficient.

It is still an object of the present invention to provide a disposable staple-feeding and forming apparatus wherein the staples are advanced in an array with the forwardmost staple being placed into a position of intended use, the forwardmost staple then being formed by a pusher and anvil combination.

It is yet an object of the present invention to provide a staple-feeding and forming mechanism which can be incorporated in a stapling instrument to provide an extremely simple and highly reliable skin-stapling apparatus.

It is still a further object of the present invention to provide a relatively large number of skin staples in a compact feeding and forming apparatus.

According to the invention, it is proposed a stapling apparatus for applying staples, each staple including a pair of spaced-apart, parallel legs, joined by a crown, said apparatus comprising:

staple-storing means adapted for storing said staples in a sequential array, one next to the other, each of said staples being oriented in said staple-storing means in a position of storage;

exiting means for providing an exit for said staples;

delivery channel means for providing a passage for said staples to said exiting means, each of said staples being oriented in a position of use when in said delivery channel means;

urging means acting on the last staple in said array for constantly urging the staples as a total charge in a predetermined direction; and

connecting channel means providing a passage for said staples from said staple-storing means to said delivery channel means being configured and positioned relative to said staple-storing means and said delivery channel means for causing the forwardmost staple in said array to be reoriented from said position of storage to said position of use when said total charge passes through said connecting channel means under the action of said urging means, the legs of each staple, when in said position storage, defining a plane which is not parallel to a plane defined by said same staple when in said position of use, characterized in that said apparatus is a surgical stapling apparatus for applying staples to disunited skin or fascia, that the angle between the plane defined by the connecting channel means and the plane defined by the crowns of the staples when they are in said staple-storing means is an acute angle of approximately 60° and that said urging means are spring means whereby the apparatus is capable of applying staples when held at any orientation relative to gravity.

These and other characteristics of the present invention, as well as many of the attendant advantages thereof, will become more readily apparent when reference is made to the following description taken in conjunction with the accompanying drawings.

Figure 1 is a side plan view of cartridge in association with a surgical stapling instrument shown in phantom, according to the state of the art.

Figure 2 is a front-end plan view of the cartridge of Figure 1.

Figure 3 is an exploded perspective of the cartridge of Figure 1 with the compression spring and surgical staples removed.

Figure 4 is a longitudinal section of the front portion of the Figure 1 cartridge with the ejector spring removed and with the pusher bar fully advanced.

Figure 5 is a top plan view of the front portion of the Figure 1 cartridge.

Figure 6 is a transverse section taken along lines 6—6 of Figure 5.

Figure 7 is a section taken along lines 7—7 of Figure 5 and shows the pusher bar almost fully retracted.

Figure 8 is a top plan view of the front half of the cartridge of Figure 1 with the cover partially shown in phantom.

Figure 9 is a top plan view of the cartridge of Figure 8 with the cartridge cover and the staple-storing cover removed.

Figure 10 is a longitudinal section of the front portion of the Figure 1 cartridge with the ejector spring removed and with the pusher bar beginning to advance.

Figures 11 through 14 are schematic illustrations of the front portion of the cartridge of Figure 1 and are used in explaining the operation of the subject invention.

Figures 15 through 19 are schematic illustrations to show how a staple is formed when securing disunited skin.

Figure 20 is an exploded perspective of a cartridge embodying the subject invention with the compression springs and surgical staples removed.

Figure 21 is a longitudinal section of the front portion of the cartridge of Figure 20 with the addition of a staple-retaining spring structure and with the pusher bar beginning to advance.

Figure 22 is a broken top plan view of the cartridge of Figure 20 with the cover partially shown in phantom.

Figure 23 is a broken top plan view, partially in section, of the cartridge of Figure 20 with the cover removed.

Figure 24 is a section taken along lines 24—24 of Figure 23.

Figure 25 illustrates examples of surgical skin staples and a wide fascia staple for use in the present invention.

Figure 26 is a partially cut-away plan view of two touching staples used to explain the operation of the subject invention.

Figure 27 is an enlarged view of the front portion of the cartridge shown in Figure 21 with the pusher advancing the staple out of the cartridge.

Figure 28 is a longitudinal section similar to that of Figure 21 with the pusher advancing the staple through the delivery channel.

Figure 29 is a partial-schematic, longitudinal section of the front portion of the Figure 20 cartridge to show the operation of the ejector springs and the staple-retaining structure.

Figure 30 is a top plan view of the front half of the cartridge of Figure 22 with a staple shown in phantom.

Figure 31 is an exploded perspective of a wide-fascia staple cartridge embodying the subject invention with the cover and wide-fascia staples removed.

Figure 32 is a side plan view of the cartridge of Figure 31 in association with a surgical stapling instrument shown in phantom.

Figure 33 is a front end plan view of the cartridge of Figure 31.

Figure 34 is a section taken along lines 34—34 of Figure 32.

Figure 35 is a longitudinal section of the front portion of the cartridge of Figure 31 with a portion of the cover removed and with the pusher bar fully advanced.

Figure 36 is a partial longitudinal section of the front portion of the cartridge of Figure 31 with an alternative staple-retaining structure.

Figure 37 is a perspective view of the staple-retaining structure of Figure 36 in cooperation with the pusher.

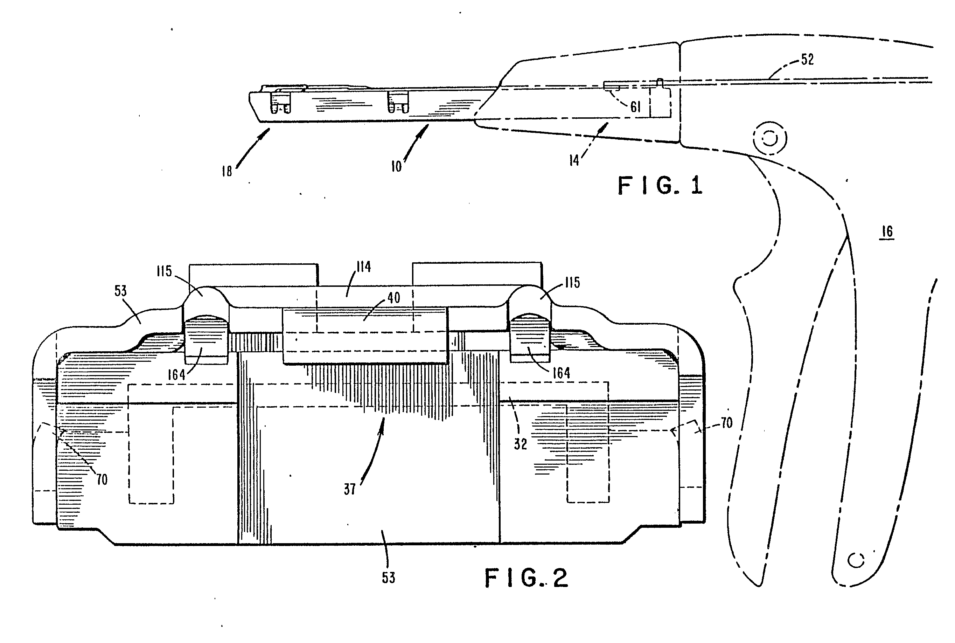

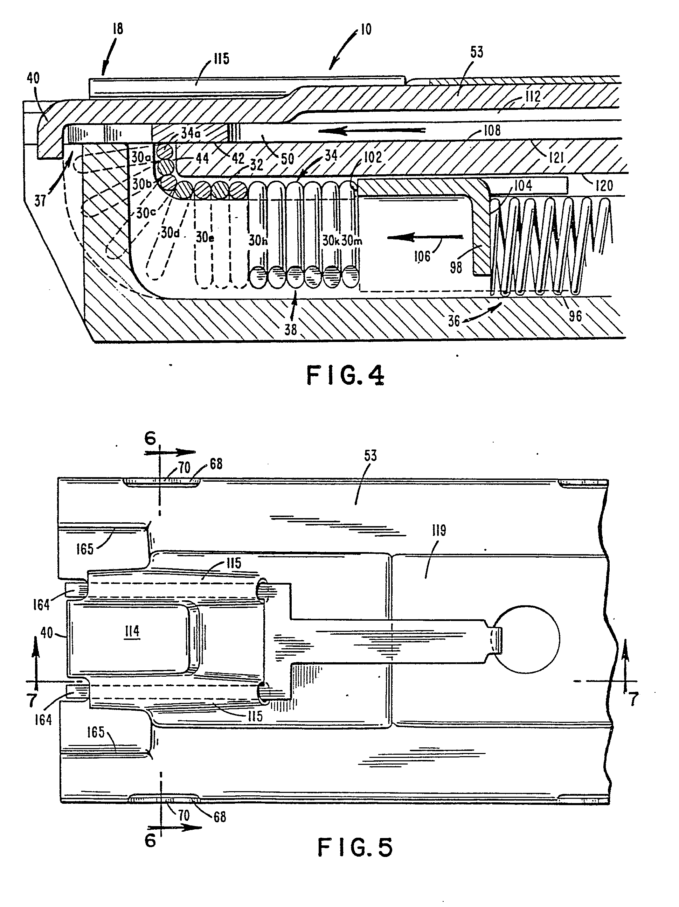

With reference to Figures 1 through 19, a known embodiment of a staple cartridge is generally designated as 10. The cartridge basically comprises a main body 12 having a rearward end 14 associating with a skin-stapling instrument 16 (a portion of which is shown in phantom) and a forward end 18 for the expulsion and formation of surgical staples. This cartridge may be associated with a permanent skin-stapling instrument, for example, those disclosed in the previously discussed prior patents, or a disposable instrument such as that disclosed in the aforementioned U.S. Patent No. 4,204,623, which is incorporated herein by reference. Defined within the main body 12 is a staple-housing or staple-storing portion 20 which is adapted to store a plurality of surgical staples 30 in a linear array 38, one next to the other. The staple-storing portion 20 contains a storage or guide track 32 for orienting the surgical staples so that the crowns or crossbars 34 (Figure 4) of the staples lie substantially in the same plane. A spring arrangement 36, contained within the main body 12, constantly urges the total charge of staples in a predetermined direction toward the forward end 18 of the cartridge. An exit aperture 37 provides a means by which the surgical staples may exit from the main body portion. Associated with the exit aperture 37 is an anvil 40 that is used to form the staples as they exit, one at a time, from the cartridge.

A delivery channel 42 contained in the main body 12 provides a passageway through which the staples move, one at a time, on their way toward the anvil. The delivery channel defines a plane spaced from and substantially parallel to a plane defined by the crowns 34 of the staples 30, as they are stored in the staple-housing portion 20. A connecting channel 44 between the storage track 32 and the delivery channel 42 provides an unobstructed path from the staple-housing portion 20 to the delivery channel for the passage of the surgical staples 30.

Mounted within the main body for rectilinear movement is a pusher 50 which responds to a force supplied by the thrust bar 52 (a portion of which is shown in phantom) of the skin-stapling instrument 16 to advance a staple through the delivery channel 42 and out of the exit aperture 37 for formation about the anvil 40.

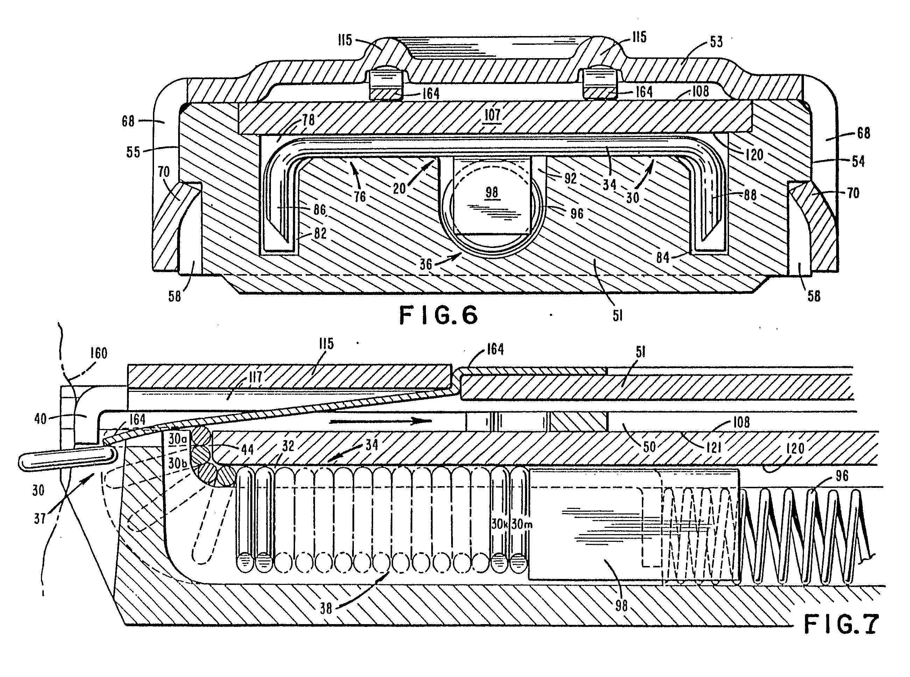

With particular reference to Figures 4 and 7, the stapling apparatus basically operates in the following manner. The staples, under the urging of the spring arrangement 36, move as an array 38 along the storage track 32 and as a total charge through the connecting channel 44 so that the forwardmost staple 30a is presented to the delivery channel 42. In this embodiment of the cartridge, the connecting channel 44 defines a plane which is substantially normal to both the plane defined by the staple crowns 34, when positioned on the storage track 32 in the staple-housing portion, and the plane defined by the delivery channel 42. Under the urging of the spring arrangement 36, the forwardmost staple 30a of the array 38 is reoriented from its storage position (see, for example, staple 30h) in the staple-housing portion to its position of intended use as it is presented to the delivery channel 42.

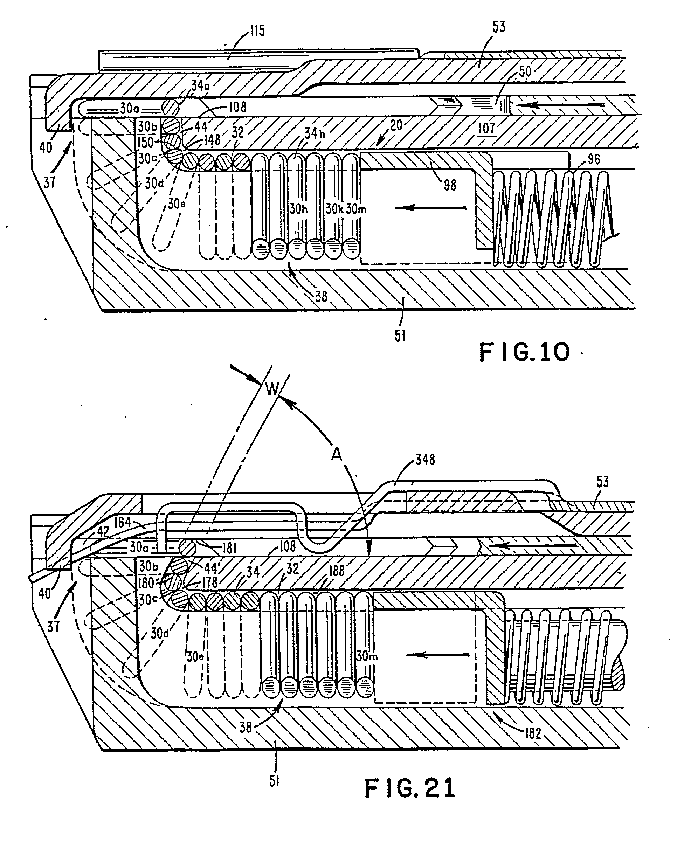

With the forwardmost staple positioned within the delivery channel, the pusher 50 is activated by a driving force supplied by the thrust bar 52 of the skin-stapling instrument 16 to move the pusher toward the forward end 18 of the cartridge (Figure 10). As it moves, the pusher 50 guides the forwardmost staple 30a through the delivery channel and out of the exit aperture 37. The forwardmost staple is then presented to the anvil 40, and, through the continued forward movement of the pusher, is then formed about the anvil.

The skin-stapling instrument 16, after completion of a stapling operation, returns the pusher to its retracted position (Figure 7), thus, permitting the next forwardmost staple 30b to be positioned within the delivery channel. With reference to Figure 4, note that the topmost staple 30a in connecting channel 44 is biased against pusher 50 by spring system 36 and the intervening staple crowns 34. Thus, during the return stroke of pusher 50, friction between the pusher and staple crown 34a will cause staple 30a to be rotated to bring the legs to their uppermost position where they lie parallel to delivery channel 42. Thus, when pusher 50 moves sufficiently to allow staple 30a to move upward into delivery channel 42, the legs of staple 30a will lie in the plane of delivery channel 42. In this way, the cartridge is ready for another stapling operation.

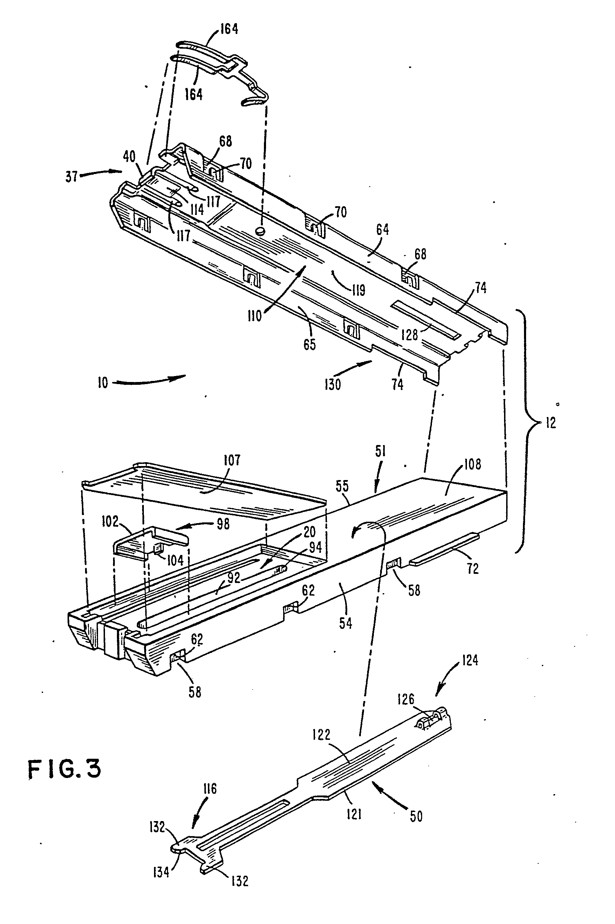

With reference to Figures 1—19, the details of the structural elements constituting the known embodiment of the cartridge will now be described. As best shown in Figure 3, the main body 12 of the cartridge 10 basically comprises an elongated body member 51, typically made of a suitable plastic such as a polycarbonate or a polystyrene, and an elongated cover 53 typically made from a suitable metal such as stainless steel. The body member 51 defines two longitudinally extending sidewalls 54 and 55, each of which contains three spaced cutouts 58 which define ledge portions 62. The cover, viewed in cross section, is generally U-shaped and includes a pair of longitudinally extending wall members 64 and 65. Each of the wall members contains three cutouts 68 which define fingers 70. In an assembled condition, the fingers 70 mate with the corresponding cutouts 58 in the side walls 54, 55 of the cartridge 10 so that the fingers press up against the ledges 62 and, thereby, secure the cover to the body member. In addition, each side wall 54, 55 of the body member 51 contains a wing portion 72 near the rear of the cartridge which mates with a corresponding cutout 74 in the sidewalls 64, 65 of the cover to further ensure proper mating of the cover and body members.

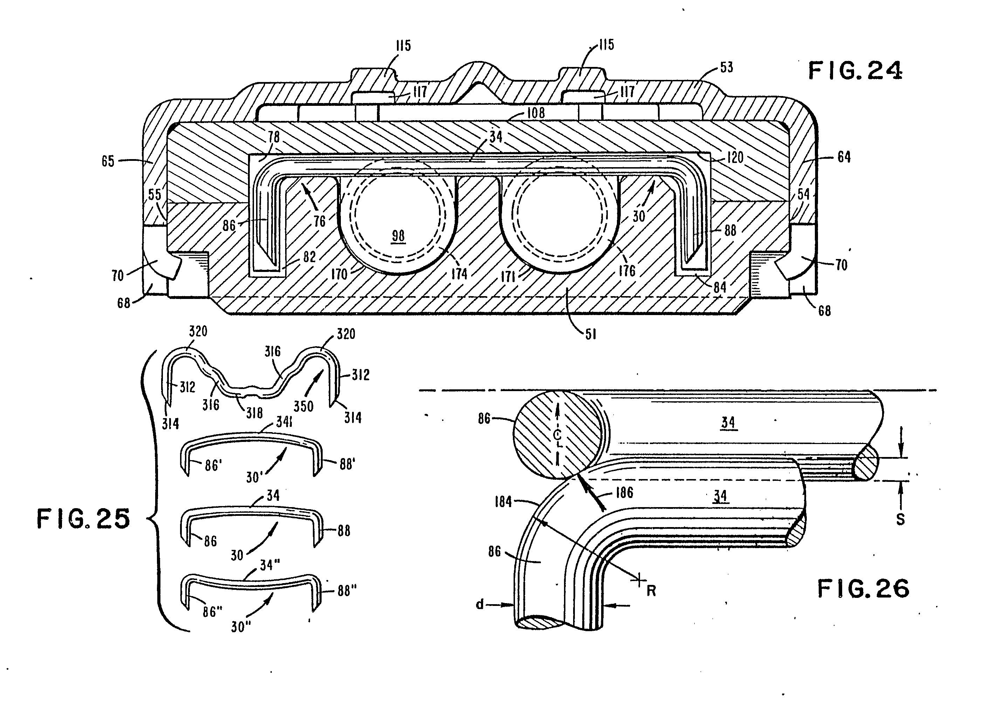

Defined within the body member 51 is the longitudinally extending staple-storing portion 20. As best seen in Figure 6, the staple-storing portion includes a storage track 76 defined by a top channel portion 78 integral with a pair of spaced apart side channels portions 82 and 84. The storage track 76, formed in part by the storage track cover 107, is dimensioned so that the staples may be received therein with the crown 34 of each staple 30 being in the top channel portion 78 and a leg 86, 88 of each staple being in one of the side channels portions 82, 84. The total charge of staples is stored within the storage track in a linear array, one next to the other. The top channel portion 78 is configured so that the crowns or crossbars 34 of the staple lie substantially in the same plane. Suitable surgical staples are illustrated in Figure 25. In one embodiment 30, the crossbar 34 is substantially straight. However, the crossbar could also bend slightly outward as in staple 30' or slightly inward as in staple 30".

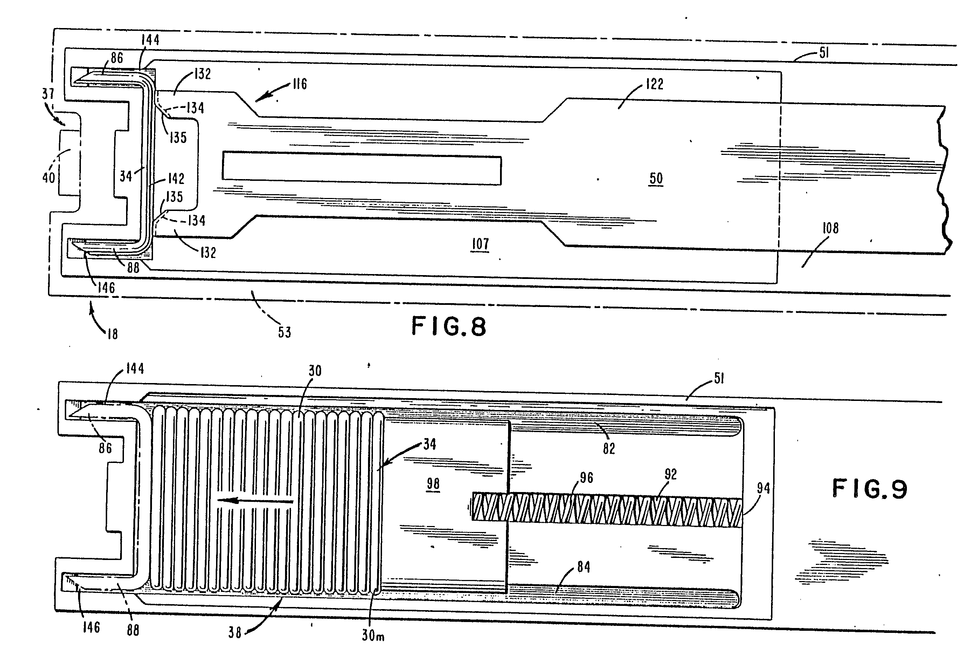

With reference to Figures 3 and 9, defined within the body member is a longitudinally extending bore 92 which is spaced between and substantially parallel to the side channel portions 82, 84 of the storage track 76. The bore terminates near the rearward portion of the body member in a transverse wall 94. Positioned within the bore is a helical compression spring 96. One end of the compression spring abuts up against the transverse wall 94, and the other end of the compression spring constantly urges a guide member 98 against the rearwardmost staple 30m of the total charge or array 38.

With reference to Figures 3—6, and 9, the guide member 98 rides in the storage track 32 and includes a staple-touching portion 102 which, when viewed in cross section, closely resembles the outline of a staple. The guide member also includes a spring-touching portion 104 which receives the expansion force of the compression spring 96. With this arrangement, the guide member 98, under the urging of the spring 96, applies a force to the rearwardmost staple 30m which in turn transfers the force to the next staple 30k, and so on, to cause the entire array of staples to move as shown by arrow 106 in a predetermined direction toward the forward end 18 of the cartridge 10.

As best illustrated in Figures 3—5, the top portion of the body member 51, with storage track cover 107 in place, defines a planar surface 108 which is spaced from and substantially parallel to the storage track 32. The top surface 108 extends throughout the entire length of the cartridge 10 and, with the interior surface 110 of the cover 53, defines the delivery channel 42 and a congruent pusher channel 112. The cover 53 contains a first raised area 114, the interior surface of which contributes in defining the delivery channel 42 and a second raised area 119, the interior surface of which contributes in defining the pusher channel 112. Raised areas 115 defines space 117 which is occupied by ejector spring 164. Shoulders 165 in cover 53 guides staple 30 in known manner as the staple emerges from the exit aperture 37. The delivery channel 42 is configured to receive a single staple in an orientation so that the crown 34 presents itself to the forward end 116 of the pusher 50 with the legs 86, 88 of the staple 30 facing the forward end 18 of the cartridge (Figure 8). The exit aperture 37 is provided at the termination point of the delivery channel 42. Also formed on the cover 54 near the termination point of the delivery channel is the anvil 40.

With continued reference to Figures 3, 4 and 8, the pusher 50 is an elongated substantially planar member which has a bottom surface 121 which rides along the top surface 108 of the body member 51 within the pusher channel 112 and a top surface 122 which presses up against the interior surface 110 of the cover 54 which defines the pusher channel. Near the rearward end 124 of the pusher 50 is a driver-receiving portion 126, which is adapted to receive the driving lug 61 of the thrust bar 52 of the skin-stapling instrument. A longitudinally extending rectangular cutout 128 in the rearward portion 130 of the cover 54 provides access to the driver portion 126, which engages the thrust bar of the skin-stapling instrument when the cartridge 10 is positioned therein.

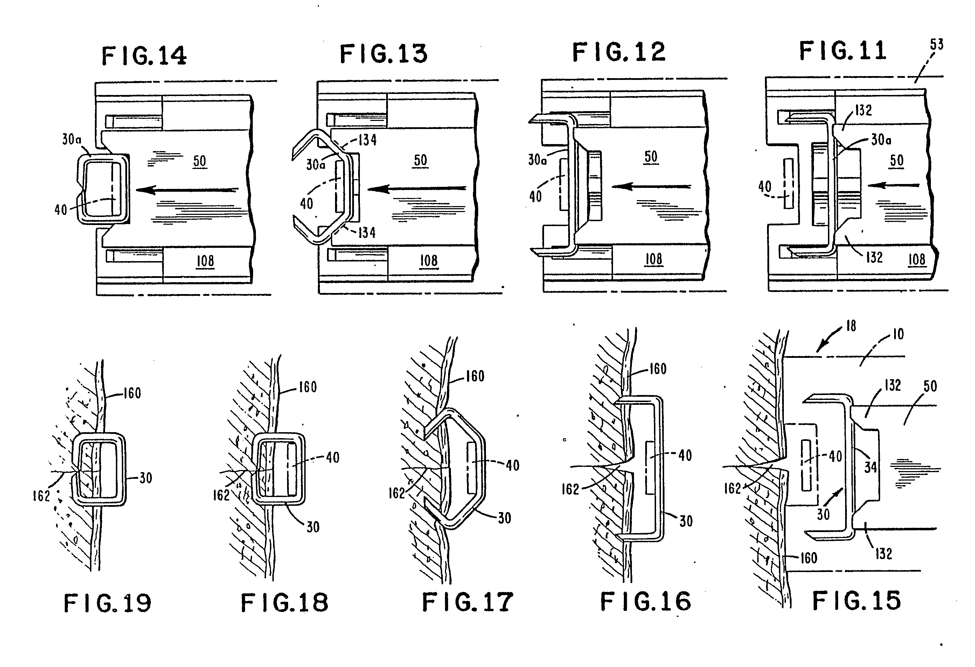

The forwardmost region 116 of the pusher element 50 is generally U-shaped, defining a pair of tines 132. As best seen in Figures 3 and 6, the tines define V-shaped grooves 134 as chamfered edges 135 to best guide the staples during the staple-discharging operation, for reasons which are explained below. With reference to Figures 11—19, the tines 132 of the pusher element 50 are spaced apart a distance designed to properly form the staple 30 around the anvil 40. More particularly, the distance between the tines is nominally equal to the width of the anvil plus twice the diameter of the staple wire.

Connecting with the termination point of the storage track 32 is the connecting channel 44 which terminates at the delivery channel 42. The connecting channel provides an unobstructed path for staples to pass from the staple-storing portion 20 to the delivery channel 42. The connecting channel 44 includes a crown-guiding portion 142 through which the crowns 34 of the various staples are guided during their passage to the delivery channel. The connecting channel also includes side passages 144, 146 which allow free movement of the legs 86, 88 of the staples during their passage to the delivery channel.

In this embodiment, as shown in Figure 10, the dimensions of the crown-guiding portion 142 are such that the staples are free to move as a total charge therein. The connecting channel 44 comprises a curved portion 148 wherein the staples are received as they exit the staple-storing portion 20, and a substantially straight portion 150 which leads to the delivery channel 42. As can be seen, the plane defined by the straight portion 150 of the connecting channel 44 is substantially perpendicular to the plane defined by the crowns 34 of the staples 30 when in the staple-storing portion, and is substantially perpendicular to the plane defined by the delivery channel 42. In turn, the plane defined by the crowns 34 of the staples 30 when in the staple-storing portion is substantially parallel to the plane defined by the delivery channel.

With reference to Figures 11—14, the operation of the stapling apparatus in the cartridge will now be described. Prior to the initial stapling operation, the pusher element 50 is located so that its forward end 116 is rearward of topmost staple 30a (Figure 10). With the pusher in this position, the cartridge is inserted into the skin-stapling instrument 16, in the case of a disposable cartridge, at which time the driving lug 61 of the thrust bar 52 engages the driver-receiving portion 126 of the pusher (Figure 1).

The forwardmost staple 30a is in the delivery channel under the urging of the spring arrangement 36. With the staple in this position, the skin-stapling instrument 16 is activated so that the thrust bar 52 causes the pusher 50 to drive the staple to and around the anvil 40 as shown in Figures 11—14.

The pusher element 50 moves forward from the position shown in Figures 10 and 11 until the staple 30a contacts the anvil 40 (Figure 12), the staple being guided by the V-shaped surface 134 of the pusher tine 142. As oriented in the figure, the pusher is driven to the left; and, because of the chamfers 135 in the tines 132, staple 30a is guided while being bent around the anvil. After the pusher element has advanced slightly, the staple takes the shape shown in Figure 12, still being guided by the V-shaped grooves 134 of the pusher tine. The chamfer surface 135 allows a bending force to be exerted on the staple at the points remote from the anvil 40. In this manner, the required initial bending force is reduced.

Once the staple is in the position shown in Figure 14, the bending force is moved from the outermost region of the chamfer to the innermost region. However, because of the buildup of inertial forces, bending is still easily accomplished. The pusher element 50 advances until it reaches the position shown in Figure 14. In this position, the staple is substantially rectangular and has its points in close proximity. Upon completion of a staple formation as shown in Figure 7, the ejector springs 164 conveniently release the staple in a known manner.

With reference now to Figures 15—19, the formation of the staple with respect to the skin will be explained. The external skin of the patient is shown at 160 and is split at an incision 162. The function of the cartridge 10 in association with the skin-stapler 16 is to securely fasten two segments of skin and to maintain the fastened position, neatly and securely, to facilitate the healing process.

As seen in Figure 15, the front end 18 of the cartridge 10 is held against the skin 160 of the patient, covering the incision. When properly positioned, the anvil 40 is centred with respect to the incision 162. The thrust bar 52 of the skin-stapler 16 is activated, and the pusher element 50 begins its forward stroke. When the pusher element 50 reaches the contact area, the forward staple 30a is ready to be ejected. The pusher contacts the first staple, as shown in Figure 15. The pusher element then expels the staple from the cartridge through the delivery channel 42 and drives same until it contacts the anvil 40, as shown in Figure 16. In this figure, it is seen that the staple pierces the skin of the patient.

In Figure 17, the staple is shown beginning to gather the skin. In this position, the staple is formed as described above with respect to Figure 13.

The staple is then completely formed around the anvil 40 so that it takes the shape illustrated in Figure 18. The corresponding position of the staple with respect to the cartridge is shown in Figure 14. During the forward thrust of the pusher element 50, the tines 132 move the pair of spring fingers 164 out of the staple plane. After the stapling operation is complete and the pusher begins to return to its rear position (Figure 7), springs 164, associated with the anvil 40, serve to eject the staple from the anvil. The skin is thus fastened and is free to expand toward the crossbar 34 of the staple (Figure 19), as may happen during postoperative swelling of the tissue.

As illustrated in Figures 9 and 10, the spring actuated guide member 98 will not turn or flex to enter the connecting channel 44 when the supply of staples is depleted. Accordingly, the last several staples occupying the connecting channel 44 will not be fed to the delivery channel. This is of minor practical significance. One could load several dummy staples in the last positions so that all "true" staples are fed and expelled. However, the staples are inexpensive and thus make their own most economical filler material. In addition, at least 50 and conceivably as many as 85 staples can be loaded in this structure, so that the few staples which cannot be expelled present no disadvantage whatsoever.

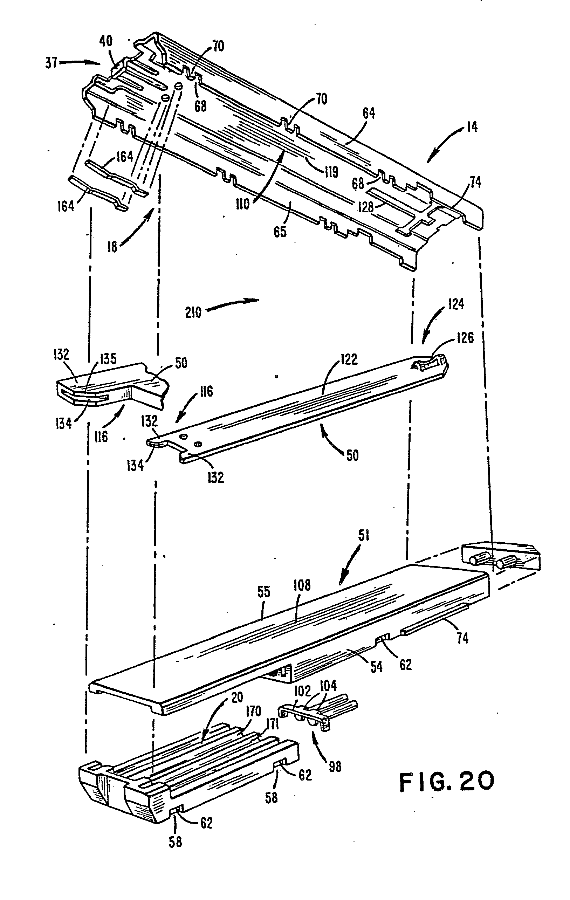

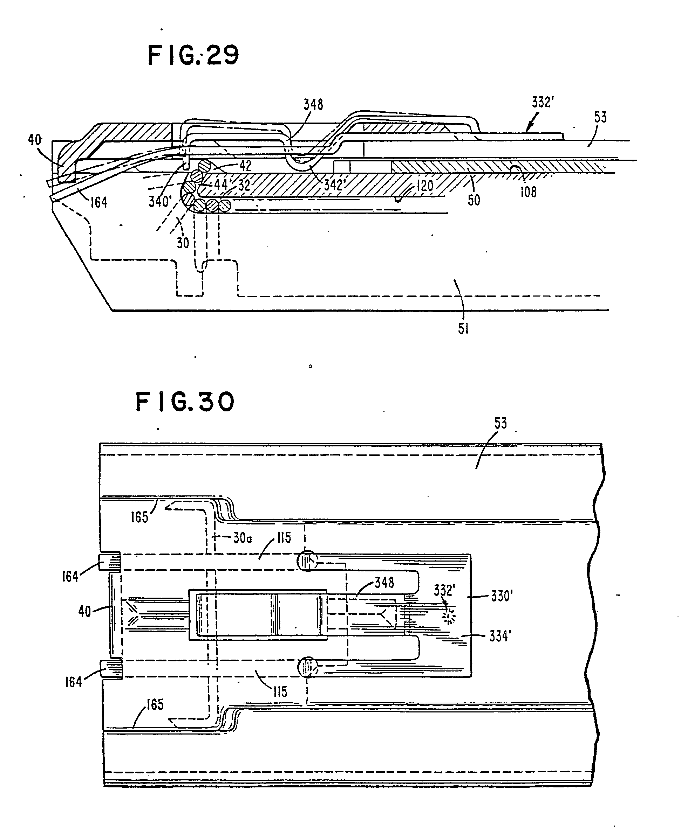

With reference to Figures 20—30, a first preferred embodiment 210 of the subject invention will now be described. Elements similar to those previously described bear the same reference numerals, and only the differences will be discussed.

This first embodiment is basically similar to the known embodiment in the provision of a staple storing portion 20, a delivery channel 42, a pusher channel 112 with pusher 50, and a connecting channel 44'. The major differences between the two embodiments are the spring arrangement 182, and the positioning and configuration of the connecting channel 44'.

As shown in Figures 20, 23 and 24, a pair of bores 170 and 171 replaces the single bore 92 of the known embodiment. Contained within each of these bores is a helical compression spring 174, 176. By this arrangement, a more uniform force may be applied to the array 38 as the staples are ejected from the cartridge 210 and formed about the anvil 40.

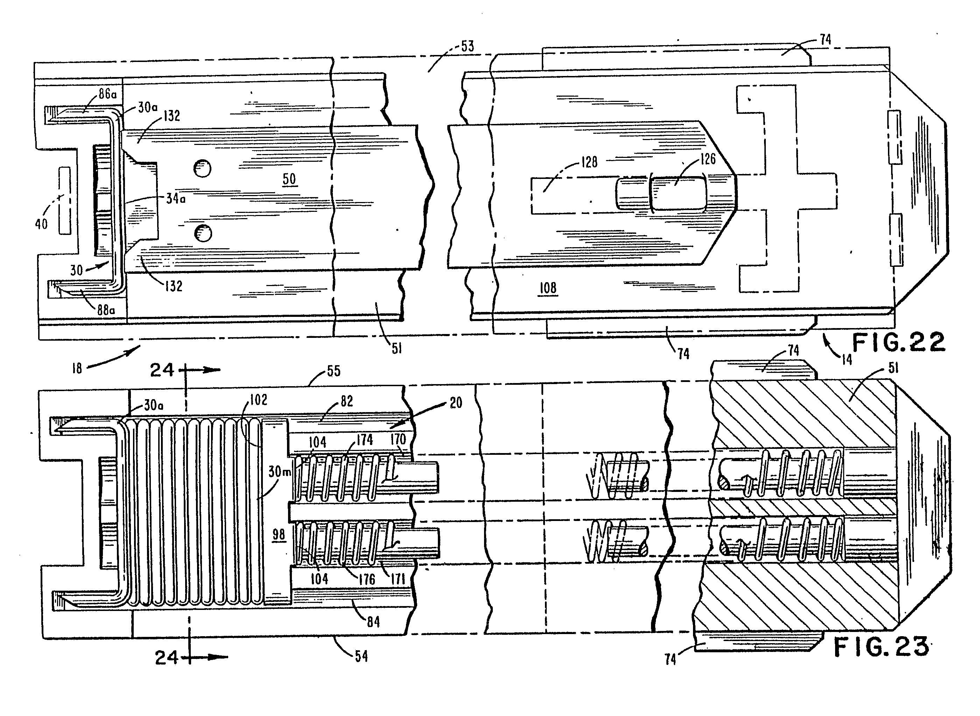

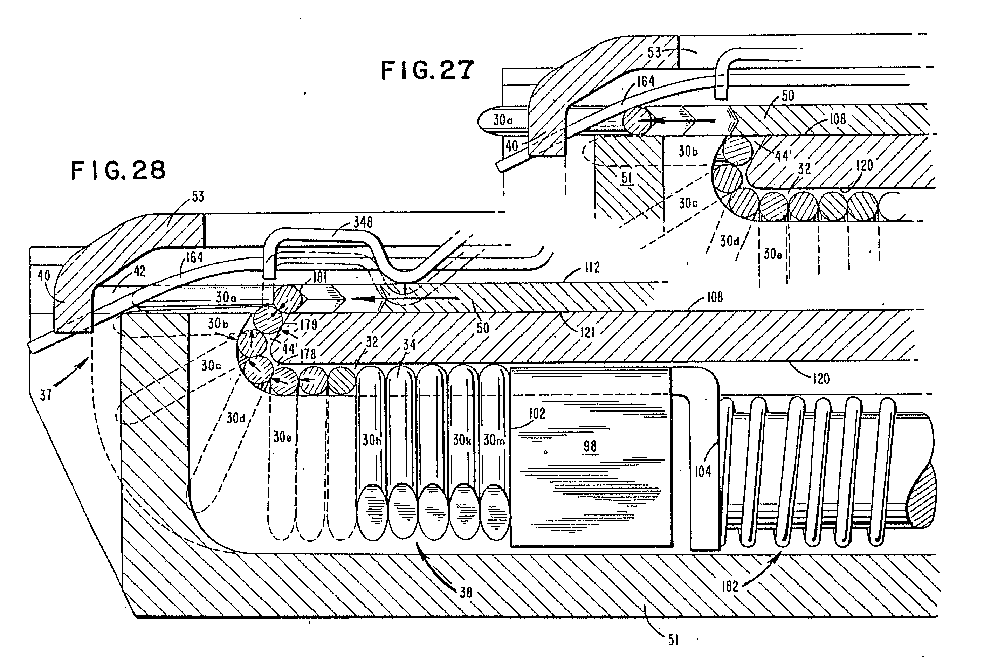

In the embodiment of the invention as illustrated in Figure 21, the connecting channel 44' forms an elbow bend 178 with the storage track 32 so that the angle A measured between the plane defined by the connecting channel and the plane defined by the crowns 34 of the staples when they are on the storage track is approximately 60 degrees. That the angle is acute is very important as hereafter explained. With reference to Figure 26, which shows the two forwardmost staples of Figure 21, the actual magnitude of the angle is determined by the geometry of the staple bend radius R relative to staple wire diameter d and width W of connecting channel 44', shown in Figure 21. It is preferred that each succeedingly higher staple in connecting track 44' be allowed to move rearwardly a distance sufficient that the lower of each pair of adjacent staples is urged upward so that its crown 34 slides against the crown of the upper staple until the outer radius R of the lower staple presses against the root of the leg section 86 of the upper staple.

In Figure 21, it has been found that, when angle A is 60° and two staples 30b and 30c occupy the connecting channel 44', staple 30b will be forceably rotated to be approximately parallel to the delivery channel 42. This ensures that staple 30a, in the delivery channel, will be held in the desired position ready for movement toward the anvil 40. That connecting channel 44' is wider than the diameter d of the staple wire is necessary for free movement of the staples. The clearance between the staple crown 34 and the walls of channel 44' allows the crowns to move in a favorable relationship one to another to cause the rotation of the sequential staples to arrive at position 30a in the desired orientation. In addition, the straight portion 180 of the connecting channel is dimensioned to be approximately equal to twice the diameter of a staple.

How the staples are moved from a position of storage within the staple storing portion 20 to a position of use within the delivery channel 42 will now be described with reference to Figures 21 and 27—29.

As shown in Figures 21 and 22, the spring arrangement 182, by applying a force against the rearwardmost staple 30m, causes the array 38 to advance as a total charge through the connecting channel 44' with the forwardmost staple 30a being placed into the delivery channel 42 in a position of use. Unlike the known embodiment, it is not necessary for the pusher 50 to be in the forward position in order to properly orient the legs 86a, 88a, of the forwardmost staple prior to its positioning within the delivery channel.

In this embodiment of the invention, because of the positioning of the connecting channel 44' relative to the storage track 32 and the delivery channel 42, the staples interact with each other to accomplish the reorientation of the forwardmost staple from its position of storage to its position of use. With reference to Figures 26 and 28, the centerline of staple 30b is offset ahead of the centerline of forwardmost staple 30a in accordance with how much the corners 184 of the staples deviate from the theoretical "mitered" shape. When the channel angle is approximately 60 degrees, the outside corner 186 of the elbow bend 184 of the staple should preferably have a radius about equal to half the staple diameter: it has been found that the combined effect of the channel angle of approximately 60° and said staple bend radius/staple diameter ratio provides an easy and accurate reorientation of the forwardmost staple from its position of storage to its position of use, as hereafter explained.

With reference to Figure 28, in the array 38 under the urging of the spring arrangement 182, staple 30e acts on staple 30d to push the crossbar 34 of staple 30d around the bend 178. Staple 30d then acts on staple 30c in the manner described above and as shown in Figure 26 with staple 30c doing the same to staple 30b, and staple 30b finally rotating staple 30a clockwise. The interaction between staples 30d, 30c and 30b is such that staple 30c is forced to the outside bend 178 of the connecting channel 44' and at the same time, staple 30b is urged toward the inside of the bend at 179. Similarly, staple 30a is urged rearwardly against guide surface 181. With this arrangement, staple 30b pushes upward against staple 30a to rotate staple 30a in a clockwise direction so that the legs 86a, 88a of the staple are held upward against the top surface of the delivery channel 42.

As best shown in Figure 21, the distance between the top surface 188 of the storage track 32 and the bottom surface 108 0f the delivery channel 42 is preferably of sufficient size to accommodate two staples in the straight portion 180 of the connecting channel 44', which is bent back at an angle of approximately 60 degrees. This particular geometry has been found advantageous to cause the legs 86b, 88b of staple 30b to be essentially parallel to the plane defined by the delivery channel 42. Accordingly, as staple 30a is pushed forward by the pusher 50 (Figures 27 and 28), the bend portions 184 of staple 30b hold staple 30a in a position parallel to the plane defined by the delivery channel 42. In this way, the legs 86a, 88a of staple 30a cannot drop downward out of the plane of the delivery channel 42, thereby leading to a staple jam.

Once the forwardmost staple 30a is positioned within the delivery channel 42, the ejection and formation of this staple about the anvil 40 under the control of the pusher 50 is the same as the first embodiment. Note, however, that, as staple 30a is pushed forward in the delivery channel 42, all of the the remaining staples in the array 38 are forced backward a small distance "s" against the spring arrangement 180. The staples are held thus until the pusher is retracted at the completion of a staple-forming cycle.

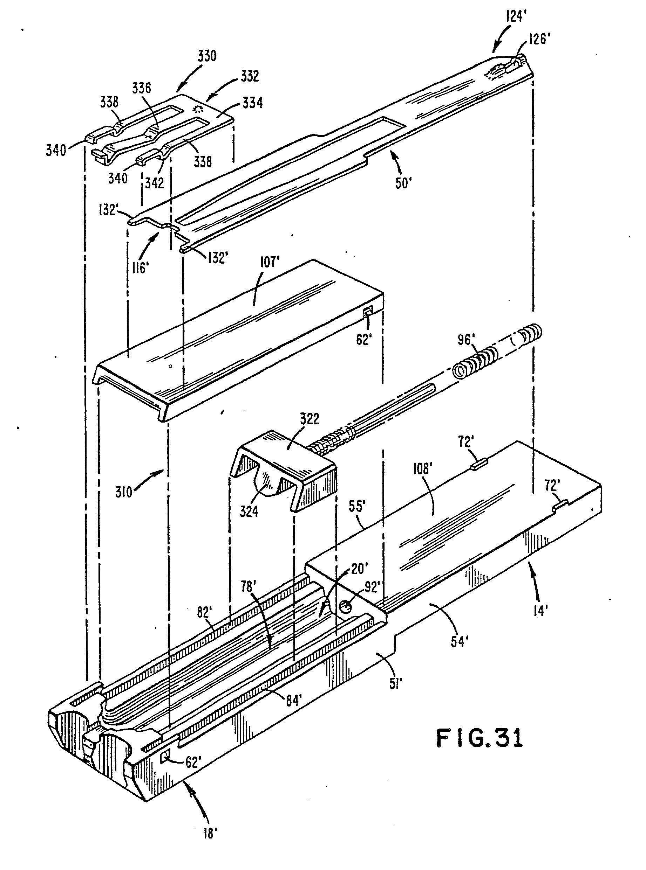

With reference to Figures 31 through 35, a second preferred embodiment of the subject invention will now be described. Elements similar to those previously described bear primed reference numerals, and only the differences will be discussed. The second embodiment of the staple-feeding apparatus is similar to the first one except that it provides for the use of a wide fascia staple 350 such as that shown in Figure 25 rather than a skin staple 30 such as that shown in the same Figure. This second embodiment basically operates in the same manner as the previously described embodiment of the invention, but contains different storage track and connection channel structures configured to accommodate the wide fascia staples. In addition, this embodiment contains a staple-retaining spring structure 330 to prevent premature ejection of a staple.

Like the first embodiment, the staple feed and ejection apparatus of the second embodiment may be incorporated into a disposable staple-carrying cartridge used with a permanent instrument or may be incorporated directly into a disposable instrument which includes the staple-carrying cartridge capabilities. Thus, as before, it can be seen that the term cartridge as used herein is meant to include both a separate structure adapted to be mounted on an instrument and a structure formed as an integral part of an instrument.

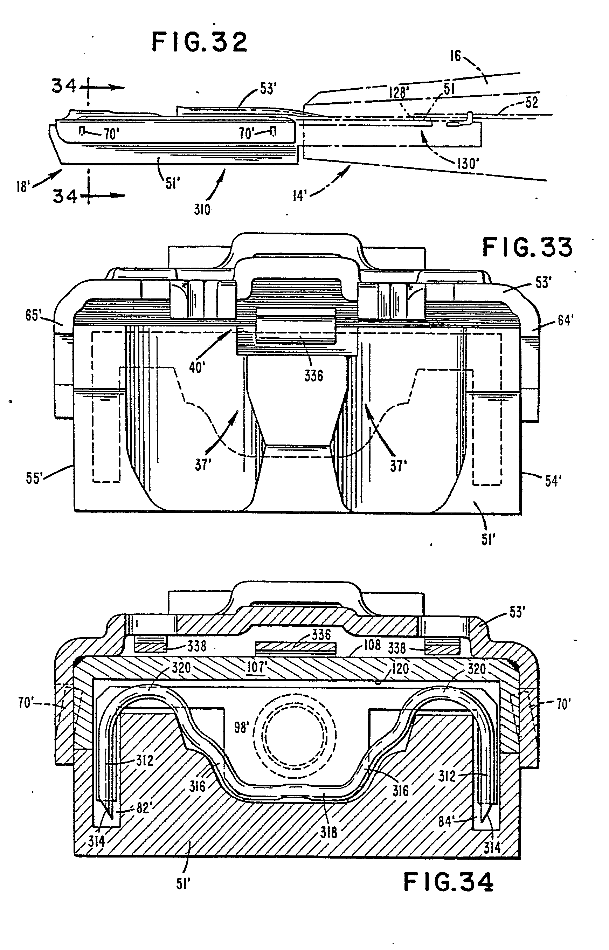

For purposes of this disclosure, the second embodiment will be described in the context of a cartridge generally shown as 310 in Figures 31 and 32 with the cartridge being mounted on a surgical stapling instrument shown in phantom in Figure 32. The surgical stapling instrument may be any conventional instrument having rectilinear thrust capabilities, similar to those previously set forth with respect to the first embodiment.

As shown in Figures 31, 32 and 34, the cartridge 310 basically comprises an elongated body member 51' that defines two longitudinally extending sidewalls 54' and 55' each of which contains a cutout which defines ledge portions 62'. In addition, each sidewall 54', 55' of the body member 51' contains an upright portion 72' near the rear of the cartridge which mates with a corresponding cutout in the sidewalls of the cover 53' to further ensure proper mating of the cover and body members. The securing of the cover 53' to the main body 51' is explained in U.S. Patent No. 4,127,227, which is incorporated by reference herein.

Defined within the body member 51' is a longitudinally extending staple storing portion 20'. Cover 107' is secured within the body 51' to cover the staple storing portion 20' in a suitable manner, such as by force fit. As best seen in Figure 34, the staple-storing portion includes a storage track 76' defined by a top channel portion 78' integral with a pair of spaced apart side channel portions 82' and 84'. The top channel portion 78' is configured to generally correspond with the structure associated with the wide fascia staple 350. As shown in Figure 34, the staple 350 has a pair of arms 312 terminating in points 314. The arms 312 occupy the side channel portion 82' and 84' of the staple storing portion 20'. The staples 350 also have crossed pieces 316 which include transverse center sections 318 which extend transversely of the arms 312. Connecting the arms 312 and the transverse center sections 318 are two arcuate sections 320 which define the crowns of the staple.

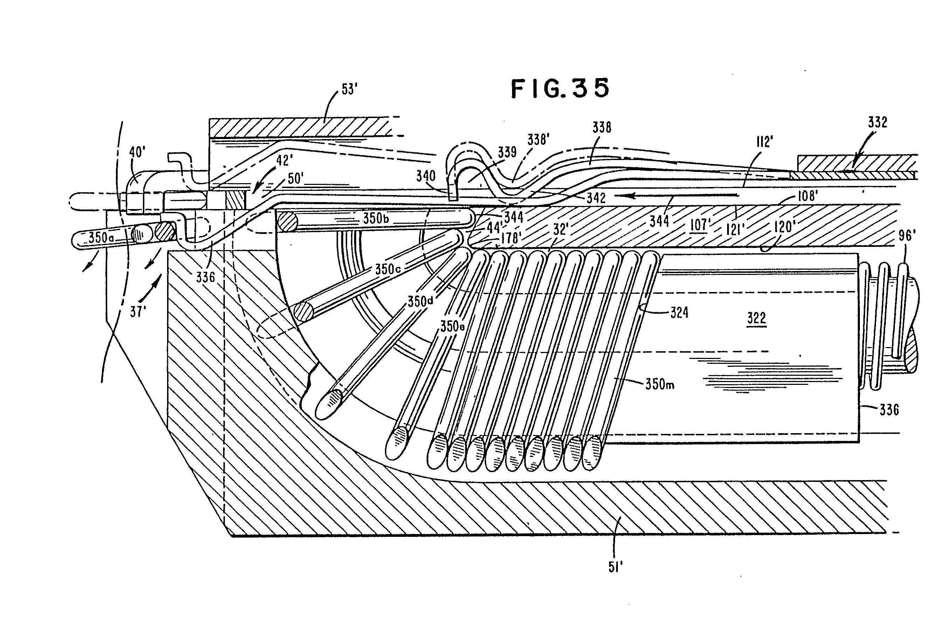

With reference to Figure 35, the total charge of staples 38' is stored within the storage track in a sequential array, one next to the other. The top channel portion 78' is configured so that the crowns 320 of the staples lie substantially in the same plane. Defined within the body member, a longitudinally extending bore 92' which is spaced between and substantially parallel to the side channel portions 82', 84' of the storage track 76'. Positioned within the bore is a helical compression spring 96'. One end of the compression spring abuts up against the termination wall of the bore 92', and the other end of the compression spring constantly urges a guide member 322 against the rearwardmost staple 30m of the total charge or array 38'. It should be understood that the two-spring arrangement could also be advantageously employed.

The guide member 322 rides in the storage track 32' and includes a staple-touching portion 324 which, when viewed in cross section, closely resembles the outline of a staple. The guide member also includes a staple-touching portion 326 which receives the expansion force of the compression spring 96'. The staple-touching portion 322 is at an incline to facilitate movement of the staples around the transition corner between the storage track 32' and the delivery channel 44'.

The top portion of the body member 51' defines a planar surface 108' which is spaced from and substantially parallel to the storage track 32'. The top surface 108' extends throughout the entire length of the cartridge 310 and, with the interior surface of the cover 53', defines the delivery channel 42' and a congruent pusher channel 112'. The cover 53', is similar to that shown in aforementioned U.S. Patent No. 4,127,227, and will not be described in detail. For purposes of this disclosure, the only requirement is that the cover, in cooperation with the top surface 108' of the body member, provide sufficient space to allow free rectilinear movement of a pusher member 50'.

The delivery channel 42' is configured to receive a single staple in an orientation so that the crowns 320 present themselves to the forward end 116' of the pusher 50' with the legs 312 of the staple 350 facing the forward end 18 of the cartridge. The exit aperture 37' is provided at the termination point of the delivery channel 42'. Also formed on the cover 53' near the termination point of the delivery channel is the anvil 40'. In this embodiment, the anvil structure, as well as the exit aperture structure, is similar to that shown in U.S. Patent No. 4,127,227 and will not be described in detail.

As best illustrated in Figures 31 and 35, the pusher 50' is an elongated substantially planar member dimensioned for free rectilinear movement within the pusher channel 112' and the congruent delivery channel 42'. Near the rearward end 124' of the pusher 50' is a driver-receiving portion 126' which is adapted to receive the driving lug 61 of the thrust bar 52 of the skin stapling instrument 16. A longitudinally extending cutout 128' in the rearward portion 130' of the cover 53' provides access to the driver portion 126', which engages the thrust bar of the skin stapling instrument when the cartridge 310 is positioned therein. The particular configuration of the forwardmost region 116' of the pusher element 50' is described in detail in aforementioned U.S. Patent No. 4,127,227.

Connecting with the termination point of the storage track 32' is the connecting channel 44' which terminates at the delivery channel 42'. The connecting channel provides an unobstructed path for staples to pass from the staple-storing portion 20' to the delivery channel 42'. The connecting channel 44' forms an elbow bend 178' with the storage track 32' so that the angle measured between the plane defined by the connecting channel and the plane defined by the crowns 320 of the staples when they are on the storage track is approximately 60°. The importance of this angle has already been described with reference to the first embodiment.

The operation of the second embodiment, in order to move staples from a position of storage within the staple-storing portion 20' to a position of use within the delivery channel 42', is substantially the same as has been previously described with respect to the first embodiment.

In order to prevent premature ejection of the staples, the cartridge 310 contains a member 330 which is secured to the cover portion 53 by suitable means such as spot welding at point 332. The member 330 includes a transverse rear section 334 from which depend a center ejection spring finger 336 and a pair of resilient staple-retaining fingers 338. The ejection spring finger 336 operates in the manner similar to that described in U.S. Patent No. 4,127,227.

With reference to Figure 35, the operation of the staple-retaining fingers 338 will be described. With the pusher 50' in its retracted position, the spring fingers 338 assume the position shown in phantom so that a downwardly projecting leg portion 340 of the spring finger is positioned in front of the crown of the staple when the staple is in the delivery channel 42'. As the pusher is advanced in the direction indicated by the arrow 344, the front portion of the pusher engages a bend 342 in the spring finger 338. The bend 342 and the leg portion 340 define a staple crown receiving area 339 which limits the forward and rearward movement of the staple 350 within the delivery channel 42', thus preventing the next forwardmost staple from entering the delivery channel while it is still occupied by a staple. As the pusher drives forward, the spring finger is deflected in an upward direction so that it assumes the position indicated by 338'. As the pusher continues its forward motion, the pusher engages the crown of the staple 350 and causes the staple to travel in the delivery channel 42' toward the exit aperture 37' for formation about the anvil 40'. In this embodiment, there is a staple-retaining spring finger 338 associated with each of the staple crowns 320. This feature could also be incorporated into the other embodiments. See, for example, Figures 27 and 28 which contain a single spring 348 which is centrally located to associate with the crown 34 of staple 30.

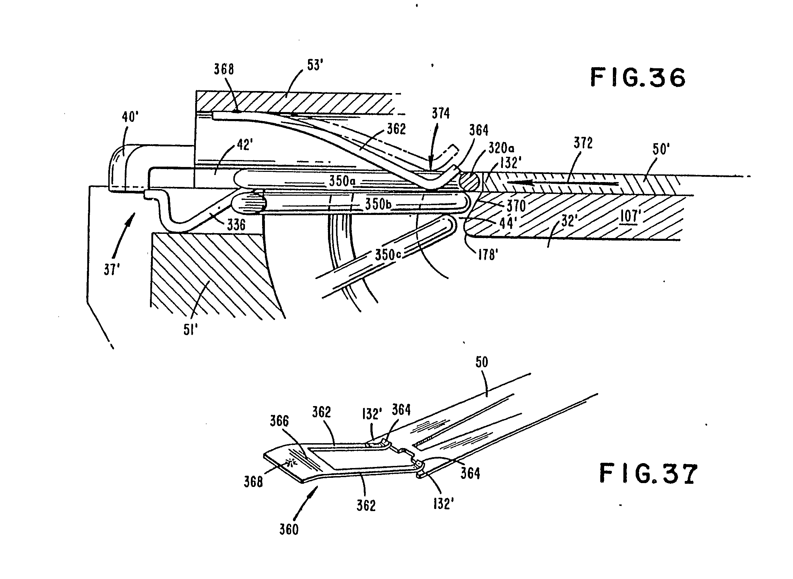

With reference to Figures 36 and 37, an alternative arrangement for the staple-retaining structure will be described. The alternative staple-retaining structure, generally designated as 360, includes a pair of resilient staple-retaining fingers 362 which each terminate in an upward bend to define a leg 364. The other ends of the spring fingers are joined by a transverse section 366.

The spring-retaining member 360 is mounted near the forward portion of the cover 53' of the cartridge by a suitable means such as spot welding at point 368.

In order to incorporate the staple-retaining member 360 into the cartridge shown in Figure 31, member 330 is altered to eliminate the spring-retaining fingers 338 and the transverse section 334, so that all that remains is the ejection spring finger 336 which is secured to the cover portion 53' by convenient means, such as spot welding, at point 332 in the same manner as previously described. Once again, the ejection spring finger 336 operates in the manner similar to that described in U.S. Patent No. 4,127,227.

With reference to Figure 35, the operation of the staple-retaining fingers 362 will be described. In this embodiment, the pusher 50' has a shorter stroke; therefore, its retracted position is such that the ends of the tines 132' line up fairly close to the rearward straight portion 370 of the connecting channel 44'. With the pusher 50' in its retracted position, the resilient fingers 362 assume the position shown so that the upwardly-turned leg portions 364 and the fingers 362 are positioned in front of the crowns 320a of the staple 350a when the staple is in the delivery channel 42'. In this way, forward movement of the staple within the cartridge is prevented by the leg portions 364, and rearward movement of the staple within the cartridge is prevented by the end of the tines 132' of the pusher 50'.

As the pusher is advanced in the direction indicated by arrow 372, the front portion of the pusher engages the crowns 320a of the staple 350a in order to start the forward motion of the staple within the delivery channel 42'. As the staple advances, the crowns engage the legs 364 of the fingers 362, causing the fingers to be deflected in an upward direction, thus clearing the path for further forward movement of the staple. As the pusher 50' continues to advance the staple, a point is reached where the staple-retaining fingers 362, at point 374, ride along the top surface of the pusher 50'. In this alternative arrangement, there is a staple-retaining finger 362 associated with each of the staple crowns 320.

Although the present invention has been shown and described in terms of specific preferred embodiments, it will be appreciated by those skilled in the art that changes or modifications are possible. For instance, while the above describes the inventive staple storing, feeding and forming mechanisms embodied as being a cartridge distinct from the powering stapling instrument, a construction where the inventive mechanism is an integral part of the stapling apparatus, i.e., there would be no cartridge as such is also to be contemplated.

## Claims

1. A stapling apparatus for applying staples, each staple including a pair of spaced-apart, parallel legs, joined by a crown, said apparatus comprising:
   staple-storing means (32) adapted for storing said staples in a sequential array, one next to the other, each of said staples being oriented in said staple-storing means in a position of storage;
   exiting means (37) for providing an exit for said staples;
   delivery channel means (42) for providing a passage for said staples to said exiting means (37), each of said staples being oriented in a position of use when in said delivery channel means;
   urging means acting on the last staple in said array for constantly urging the staples as a total charge in a predetermined direction; and
   connecting channel means (44') providing a passage for said staples from said staple-storing means to said delivery channel means, said connecting channel means being configured and positioned relative to said staple-storing means and said delivery channel means for causing the forwardmost staple in said array to be reoriented from said position of storage to said position of use when said total charge passes through said connecting channel means under the action of said urging means, the legs of each staple, when in said position storage, defining a plane which is not parallel to a plane defined by said same staple when in said position of use, characterized in that said apparatus is a surgical stapling apparatus for applying staples to disunited skin or fascia, that the angle (A) between the plane defined by the connecting channel means (44') and the plane defined by the crowns (34) of the staples when they are in said staple-storing means (32) is an acute angle of approximately 60° and that said urging means are spring means (182) whereby the apparatus is capable of applying staples when held at any orientation relative to gravity.

2. The apparatus of claim 1, further comprising anvil means (40) for forming said staples passing through said exiting means (37).

3. The apparatus of claim 2, further comprising pusher means (50) for pushing said staples through said delivery channel means (42) and out of said exiting means (37) for formation about said anvil means (40).

4. The apparatus of claim 3, wherein said delivery channel means for accommodating one staple at a time, said one staple being received from said connecting channel means (44').

5. The apparatus of claim 1, wherein said staple-storing means (32) includes means for orienting said staple in said array so that the crowns of said staples lie substantially in the same plane.

6. The apparatus of claim 5, wherein said delivery channel means (42) defines a plane which is substantially parallel to and spaced from the plane defined by said staple crowns when said crowns are in said staple-storing means.

7. The apparatus of claim 1, for applying staples the elbow bend radius (R) of which is about equal to half the staple diameter (d), wherein the staples in said connecting channel means (44') interact with each other by friction under the urging of the spring means (182), the combined effect of the channel angle of approximately 60° and said R/d ratio being that each staple, when in said connecting channel, imparts rotational movement on the next succeeding staple, said rotational movement being about the axis defined by the crown of the staple, with the forwardmost staple of said array being finally positioned for use in said delivery channel (42), so that an easy and accurate reorientation of the forwardmost staple from its position of storage to its position of use is obtained.

## Patentansprüche

1. Heftvorrichtung zum Einsetzen von Klammern, wobei jede Klammer ein Paar von voneinander beabstandeten parallelen Schenkeln aufweist, welche über ein Querstück verbunden sind, folgendes aufweisend:

— eine Klammernmagaziniereinrichtung (32), welche für ein Magazinieren der Klammern in einer Reihenfolge, bei welcher eine Klammer an der anderen anliegt und jede Klammer in der Klammermagaziniereinrichtung in einer Lagerstellung ausgerichtet ist, ausgelegt ist;

— eine Auslaßeinrichtung (37), welche den Austritt der Klammern sicherstellt;

— einen Förderkanal (42), welcher für die Klammern einen Durchgang zur Auslaßeinrichtung (37) bildet, wobei jede Klammer im Förderkanal in einer Verwendungsstellung angeordnet ist;

— eine Treibeeinrichtung, welche auf die letzte Klammer in der Reihe einwirkt und die Klammern als Gesamtladung in eine vorbestimmte Richtung treibt; und

— einen Verbindungskanal (44'), welcher einen Durchlaß für die Klammern von der Klammernmagaziniereinrichtung zum Förderkanal schafft und in bezug auf die Klammernmagaziniereinrichtung so ausgebildet und positioniert ist, daß die vorderste Klammer der Klammernreihe aus der Speicherstellung in die Gebrauchsstellung umgerichtet wird, wenn die Gesamtladung unter der Wirkung der Treibeeinrichtung durch den Verbindungskanal befördert wird, wobei die Schenkeln jeder Klammer in der Speicherstellung eine Ebene bestimmen, die nicht zu jener Ebene parallel verläuft, welche von derselben Klammer in der Gebrauchsstellung bestimmt wird, dadurch gekennzeichnet, daß die Heftvorrichtung eine chirurgische Heftvorrichtung zum Einsetzen von Klammern in getrennte Haut oder in eine Binde ist, daß der Winkel (A) zwischen der vom Verbindungskanal (44') gebildeten Ebene und jener Ebene, die von den Querstücken (34) der Klammern gebildet wird, wenn sich diese in der Klammernmagaziniereinrichtung (32) befinden, ein spitzer Winkel von etwa 60° ist und daß die Treibeeinrichtung eine Feder (182) ist, wobei die Vorrichtung beim Einsetzen von Klammern in jeder beliebigen Stellung in bezug auf die Schwerkraft gehalten werden kann.

2. Vorrichtung nach Anspruch 1, weiters gekennzeichnet durch einen Amboß (40) zum Formen der durch die Auslaßeinrichtung (37) tretenden Klammern.

3. Vorrichtung nach Anspruch 2, weiters gekennzeichnet durch einen Schieber (50), welcher die Klammern durch den Förderkanal (42) und aus der Auslaßvorrichtung (37) schiebt, damit diese über dem Amboß (40) geformt werden.

4. Vorrichtung nach Anspruch 3, dadurch gekennzeichnet, daß der Förderkanal (42) eine Einrichtung, welche eine vom Verbindungskanal (44') erhaltene Klammer zu einem Zeitpunkt aufnimmt, aufweist.

5. Vorrichtung nach Anspruch 1, dadurch gekennzeichnet, daß die Klammernmagaziniereinrichtung (32) eine Vorrichtung aufweist, welche die Klammern in der Reihenfolge so ausrichtet, daß die Querstücke der Klammern im wesentlichen in der selben Ebene liegen.

6. Vorrichtung nach Anspruch 5, dadurch gekennzeichnet, daß der Förderkanal (42) eine Ebene bestimmt, welche zu der von den Querstücken der Klammern gebildeten Ebene im wesentlichen parallel und von dieser Ebene beabstandet ist, wenn sich die Klammern in der Klammernmagaziniereinrichtung befinden.

7. Vorrichtung nach Anspruch 1 zum Einsetzen von Klammern, deren Winkelkrümmungsradius (R) etwa halb so groß wie der Klammerndurchmesser (d) ist, dadurch gekennzeichnet, daß die Klammern im Verbindungskanal (44') infolge des Antriebs durch die Feder (182) durch Reibung untereinander zusammenwirken, wobei der Kombinationseffekt des Kanalwinkels von etwa 60° und des R/d-Verhältnisses darin besteht, daß jede sich im Verbindungskanal befindende Klammer auf die nächste darauffolgende Klammer eine um die vom Querstück der Klammer bestimmte Achse verlaufende Drehbewegung ausübt, bei welcher die vorderste Klammer der Reihenfolge schließlich zum Zweck des Gebrauchs im Förderkanal (42) so positioniert wird, daß eine einfache und zuverlässige Umordnung der vordersten Klammer aus ihrer Speicherstellung in ihre Gebrauchsstellung erzielt wird.

**Revendications**

1. Agrafeuse pour poser des agrafes, chaque agrafe présentant une paire de branches parallèles et espacées, réunies par une âme, ladite agrafeuse comportant:

des moyens de stockage des agrafes (32) prévus pour stocker lesdites agrafes sous forme d'un train d'agrafes successives, l'une près de l'autre, chacune desdites agrafes étant orientée dans lesdits moyens de stockage des agrafes en position de stockage;

des moyens formant sortie (37) pour fournir une sortie pour lesdites agrafes;

des moyens formant canal d'alimentation (42) pour fournir un passage auxdites agrafes vers lesdits moyens de sortie (37), chacune desdites agrafes étant orientée dans une position d'utilisation lorsqu'elle se trouve dans lesdits moyens formant canal d'alimentation;

des moyens de poussée agissant sur la dernière agrafe dudit train pour pousser constamment les agrafes, formant une charge globale, dans une direction prédéterminée; et

des moyens formant canal de liaison (44') fournissant auxdites agrafes un passage depuis lesdits moyens de stockage des agrafes vers lesdits moyens formant canal d'alimentation, lesdits moyens formant canal de liaison ayant une forme et une position en rapport avec lesdits moyens de stockage des agrafes et avec lesdits moyens formant canal d'alimentation de façon à faire que l'agrafe la plus en avant dans ledit train soit réorientée pour passer de ladite position de stockage à la dite position de d'utilisation lorsque ladite charge globale passe à travers lesdits moyens formant canal d'alimentation sous l'action desdits moyens de poussée, les branches de chaque agrafe, lorsqu'elle se trouve dans ladite position de stockage, définissant un plan qui n'est pas parallèle au plan défini par ladite même agrafe lorsqu'elle se trouve dans ladite position d'utilisation, caractérisée en ce que ladite agrafeuse est une agrafeuse chirurgicale pour poser des agrafes sur de la peau ou sur une formation aponévrotique à lèvres ouvertes; en ce que l'angle (A) du plan défini par les moyens formant canal de liaison (44') et du plan défini par les âmes (34) des agrafes lorsqu'elles se trouvent dans lesdits moyens de stockage des agrafes (32) est un angle aigu d'environ 60°; et en ce que lesdits moyens de poussée sont des moyens formant ressort (182), ce par quoi l'agrafeuse est capable de poser des agrafes lorsqu'elle est tenue en toute orientation par rapport à la verticale.

2. Agrafeuse selon la revendication 1, comportant en outre des moyens formant enclume (40) pour donner leur forme auxdites agrafes passant à travers lesdits moyens de sortie (37).

3. Agrafeuse selon la revendication 2, comportant en outre des moyens formant poussoir (50) pour pousser lesdites agrafes à travers lesdits moyens formant canal d'alimentation (42) et hors desdits moyens de sortie (37) pour prendre leur forme autour desdits moyens formant enclume (40).

4. Agrafeuse selon la revendication 3, où lesdits moyens formant canal d'alimentation (42) comportent des moyens pour reprendre une agrafe à la fois, ladite agrafe provenant desdits moyens formant canal de liaison (44').

5. Agrafeuse selon la revendication 1, où lesdits moyens de stockage des agrafes (32) comportent des moyens pour orienter lesdites agrafes dudit train de façon que les âmes desdites agrafes soient sensiblement dans le même plan.

6. Agrafeuse selon la revendication 5, où lesdits moyens formant canal d'alimentation (42) définissent un plan qui est sensiblement parallèle au, et à une certaine distance du, plan défini par lesdites âmes des agrafes lorsque lesdites âmes sont dans lesdits moyens de stockage des agrafes.

7. Agrafeuse selon la revendication 1, pour poser des agrafes dont le rayon de courbure (R) de pliage est à peu près égal à une fois et demi le diamètre (d) de l'agrafe, étant précisé que les agrafes qui se trouvent dans lesdits moyens formant canal de liaison (44') agissent l'une sur l'autre par frottement sous la poussée des moyens formant ressort (182), l'effet combiné de l'angle du canal, d'environ 60°, et dudit rapport R/d étant que chaque agrafe, lorsqu'elle se trouve dans ledit canal de liaison, exerce un mouvement de rotation sur l'agrafe voisine, ledit mouvement de rotation ayant lieu autour de l'axe défini par l'âme de l'agrafe, l'agrafe dudit train la plus en avant étant finalement positionnée pour utilisation dans ledit canal d'alimentation (42), de sorte que l'on obtient une réorientation facile et précise de l'agrafe la plus en avant pour passer de sa position de stockage à sa position d'utilisation.

FIG. 1

FIG. 2

FIG.3

FIG.4

FIG.5

FIG.6

FIG.7

0 040 157

FIG.8

FIG.9

FIG.10

FIG.21

FIG.14  FIG.13  FIG.12  FIG.11

FIG.19  FIG.18  FIG.17  FIG.16  FIG.15

0 040 157

FIG. 20

FIG.22

FIG.23

0 040 157

FIG.24

FIG.26

FIG.25

FIG.27

FIG.28

# FIG. 29

# FIG. 30

FIG.31

# FIG.32

# FIG.33

# FIG.34

FIG. 35

0 040 157

FIG.36

FIG.37

0 040 157